# DEMANDE DE BREVET EUROPEEN

(11) **EP 2 338 468 A1**
(43) Date de publication de la demande: **29.06.2011**
(21) Numéro de dépôt: 10195449.3
(22) Date de dépôt: 16.12.2010
(51) Int. Cl.: A61K 8/31, A61K 8/41, A61K 8/42, A61K 8/81, A61Q 5/00, A61Q 5/12

(54) **Composition cosmétique comprenant au moins un alcane linéaire volatil, au moins un tensioactif cationique dans un rapport alcane(s) linéaire(s) volatil(s) tensioactif(s) cationique(s) particulier**

(30) Priorité: 23.12.2009 FR 0959549
(71) Demandeur: L'Oréal, 75008 Paris (FR)
(72) Inventeur: Desenne, Patricia, 74370, Pringy (FR); Chesneau, Laurent, 92300, Levallois (FR)
(74) Mandataire: Dossmann, Gérard

(57) **Abrégé**

La présente invention concerne une composition cosmétique comprenant dans un milieu cosmétiquement acceptable :
- au moins 2,5 % en poids d'un ou plusieurs alcanes linéaires volatils par rapport au poids total de la composition,
- un ou plusieurs tensioactifs cationiques,

le rapport pondéral alcane(s) linéaire(s) volatil(s) / tensioactif(s) cationique(s) étant inférieur ou égal à 1,5.

Elle concerne aussi son utilisation pour le traitement cosmétique des matières kératiniques, de préférence des fibres kératiniques comme les cheveux, et un procédé de traitement cosmétique des matières kératiniques mettant en oeuvre ladite composition.

## Description

La présente invention concerne une composition cosmétique comprenant un ou plusieurs alcane(s) linéaire(s) volatil(s), un ou plusieurs tensioactif(s) cationique(s), dans des proportions particulières, son utilisation pour le traitement cosmétique des matières kératiniques, de préférence des fibres kératiniques comme les cheveux et un procédé de traitement cosmétique des matières kératiniques mettant en oeuvre ladite composition.

Dans le domaine du traitement capillaire, l'utilisation de solvants volatils est connue dans des produits capillaires de soins rincés ou non rincés. Ils sont généralement utilisés pour différentes raisons. Ils permettent notamment de modifier le rendu sensoriel d'un produit capillaire en lui conférant une texture légère et non collante dans la main. Ils peuvent également lui conférer un caractère glissant qui facilite la répartition du produit sur les cheveux et en particulier sur des cheveux secs.

Dans les émulsions aqueuses de type huile-dans-eau, qui peuvent se présenter sous forme de crèmes plus ou moins gélifiées, l'ajout de solvants volatils peut également permettre de solubiliser des gommes de silicone qui, de par leur viscosité intrinsèque, seraient difficiles à introduire dans les compositions.

Ces solvants volatils qui sont généralement des esters gras liquides, des huiles hydrocarbonées de type isododécane ou isohexadécane, et/ou des huiles siliconées, peuvent notamment induire des problèmes de toucher gras, de manque de brillance, de cheveux raidis, durs.

Il subsiste donc un besoin de remplacer ces solvants volatils pour éviter les inconvénients cités ci-dessus.

La demanderesse a maintenant découvert de façon inattendue et surprenante, que l'association d'au moins un alcane linéaire volatil et d'au moins tensioactif cationique dans un rapport pondéral particulier et dans des proportions particulières permettait d'éviter les inconvénients mentionnés ci-dessus et d'améliorer les propriétés cosmétiques telles que le lissage, la souplesse, le démêlage, le volume notamment par décollement des racines, et la tonicité de la chevelure.

En particulier, la composition selon l'invention permet d'obtenir des cheveux plus lisses, plus souples, avec un comportement homogène le long du cheveu et entre cheveux, au moment du rinçage. Sur cheveux humides, on obtient des cheveux plus faciles à démêler ou plus toniques et/ou ayant des racines plus décollées (en racines, les cheveux ne sont pas plaqués sur le cuir chevelu mais forment un angle, d'où apport de volume). En outre, les cheveux secs sont plus souples et/ou plus lisses au toucher.

Ainsi, l'invention a pour objet une composition cosmétique comprenant, dans un milieu cosmétiquement acceptable :
- au moins 2,5% en poids d'un ou plusieurs alcanes linéaires volatils par rapport au poids total de la composition, et
- un ou plusieurs tensioactifs cationiques,
le rapport pondéral alcane(s) linéaire(s) volatil(s) / tensioactif(s) cationique(s) étant inférieur ou égal à 1,5.

Elle a encore pour objet l'utilisation de ladite composition pour le traitement cosmétique des matières kératiniques, de préférence des fibres kératiniques comme les cheveux, notamment comme produit capillaire de soin rincé.

Un autre objet de l'invention est un procédé de traitement cosmétique des matières kératiniques, de préférence des fibres kératiniques comme les cheveux, mettant en oeuvre ladite composition.

La composition cosmétique selon l'invention comprend, dans un milieu cosmétiquement acceptable :
- au moins 2,5% en poids d'un ou plusieurs alcanes linéaires volatils par rapport au poids total de la composition, et
- un ou plusieurs tensioactifs cationiques,
le rapport pondéral de la quantité d'alcane(s) linéaire(s) volatil(s) sur la quantité de tensioactif(s) cationique(s) étant inférieur ou égal à 1,5.

Ledit rapport va de préférence de 0,01 à 1,5, en particulier de 0,1 à 1,4, et mieux encore de 0,5 à 1,2.

Par « un ou plusieurs alcane(s) linéaire(s) volatil(s) », on entend indifféremment « une ou plusieurs huile(s) alcane(s) linéaire(s) volatile(s) ».

Un alcane linéaire volatil convenant à l'invention est liquide à température ambiante (environ 25 °C) et sous pression atmosphérique (101 325 Pa ou 760 mm Hg).

Par « alcane linéaire volatil » convenant à l'invention, on entend un alcane linéaire, susceptible de s'évaporer au contact de la peau en moins d'une heure, à température ambiante (25°C) et sous pression atmosphérique (101 325 Pa), liquide à température ambiante, ayant notamment une vitesse d'évaporation allant de 0,01 à 15 mg/cm²/min, à température ambiante (25°C) et sous pression atmosphérique (101 325 Pa).

De préférence, le ou les alcanes linéaires volatils convenant à l'invention présentent une vitesse d'évaporation allant de 0,01 à 3,5 mg/cm²/min, mieux de 0,01 à 1,5 mg/cm²/min, à température ambiante (25°C) et sous pression atmosphérique (101 325 Pa).

De façon plus préférée, le ou les alcanes linéaires volatils convenant à l'invention présentent une vitesse d'évaporation allant de 0,01 à 0,8 mg/cm²/min, préférentiellement de 0,01 à 0,3 mg/cm²/min, et encore plus préférentiellement de 0,01 à 0,12 mg/cm²/min, à température ambiante (25°C) et sous pression atmosphérique (101 325 Pa).

La vitesse d'évaporation d'un alcane volatil conforme à l'invention (et plus généralement d'un solvant volatil) peut être notamment évaluée au moyen du protocole décrit dans WO 06/013413, et plus particulièrement au moyen du protocole décrit ci-après.

On introduit dans un cristallisoir (diamètre : 7 cm) placé sur une balance se trouvant dans une enceinte d'environ 0,3 m³ régulée en température (25 °C) et en hygrométrie (humidité relative 50 %), 15 g de solvant hydrocarboné volatil.

On laisse le solvant hydrocarboné volatil s'évaporer librement, sans l'agiter, en assurant une ventilation par un ventilateur (PAPST-MOTOREN, référence 8550 N, tournant à 2700 tours/minute) disposé en position verticale au-dessus du cristallisoir contenant le solvant hydrocarboné volatil, les pales étant dirigées vers le cristallisoir, à une distance de 20 cm par rapport au fond du cristallisoir.

On mesure à intervalles de temps réguliers la masse de solvant hydrocarboné volatil restante dans le cristallisoir.

On obtient alors le profil d'évaporation du solvant en traçant la courbe de la quantité de produit évaporé (en mg/cm²) en fonction du temps (en min).

Puis on calcule la vitesse d'évaporation qui correspond à la tangente à l'origine de la courbe obtenue. Les vitesses d'évaporation sont exprimées en mg de solvant volatil évaporé par unité de surface (cm²) et par unité de temps (minute).

Selon un mode de réalisation préféré, le ou les alcanes linéaires volatils convenant à l'invention ont une pression de vapeur (appelée également pression de vapeur saturante) non nulle, à température ambiante, en particulier une pression de vapeur allant de 0,3 Pa à 6000 Pa.

De façon préférée, le ou les alcanes linéaires volatils convenant à l'invention ont une pression de vapeur allant de 0,3 à 2000 Pa, mieux de 0,3 à 1000 Pa, à température ambiante (25°C).

De façon plus préférée, le ou les alcanes linéaires volatils convenant à l'invention ont une pression de vapeur allant de 0,4 à 600 Pa, préférentiellement de 1 à 200 Pa, et encore plus préférentiellement de 3 à 60 Pa, à température ambiante (25°C).

Selon un mode de réalisation, un alcane linéaire volatil convenant à l'invention peut présenter un point éclair compris dans l'intervalle variant de 30 à 120 °C, et plus particulièrement de 40 à 100 °C. Le point éclair est en particulier mesuré selon la Norme iso 3679.

Selon un mode de réalisation, le ou les alcanes linéaires volatils convenant dans l'invention peuvent être les alcanes linéaires comportant de 7 à 15 atomes de carbone, de préférence de 8 à 14 atomes de carbone, et mieux de 9 à 14 atomes de carbone.

De façon plus préférée, le ou les alcanes linéaires volatils convenant dans l'invention comprennent de 10 à 14 atomes de carbone, et encore plus préférentiellement de 11 à 14 atomes de carbone.

Le ou les alcanes linéaires volatils convenant à l'invention peuvent être avantageusement d'origine végétale.

De préférence, l'alcane linéaire volatil ou le mélange d'alcanes linéaires volatils présent dans la composition selon l'invention comprend au moins un isotope ¹⁴C du carbone (carbone 14). En particulier, l'isotope ¹⁴C peut être présent en un ratio isotopique ¹⁴C/¹²C (en nombre d'isotopes) supérieur ou égal à 1.10⁻¹⁶, de préférence supérieur ou égal à 1.10⁻¹⁵ , de préférence encore supérieur ou égal 7,5.10⁻¹⁴, et mieux supérieur ou égal 1,5.10⁻¹³. De préférence, le ratio isotopique ¹⁴C / ¹²C va de 6.10⁻¹³ à 1,2.10⁻¹²

La quantité d'isotopes ¹⁴C dans l'alcane linéaire volatil ou le mélange d'alcanes linéaires volatils peut être déterminée par des méthodes connues de l'homme du métier telles que la méthode de comptage de Libby, la spectrométrie à scintillation liquide ou encore la spectrométrie de masse à accélération (Accelerator Mass Spectrometry).

Un tel alcane ou mélange d'alcanes peut être obtenu, directement ou en plusieurs étapes, à partir d'une matière première végétale comme une huile, un beurre, une cire, etc.

A titre d'exemple d'alcanes convenant à l'invention, on peut mentionner les alcanes décrits dans les demandes de brevets WO 2007/068371 et WO2008/155059. Ces alcanes sont obtenus à partir d'alcools gras, eux-mêmes obtenus à partir d'huile de coprah ou de palme.

A titre d'exemples d'alcanes linéaires convenant à l'invention, on peut citer le n-heptane (C7), le n-octane (C8), le n-nonane (C9), le n-décane (C10), le n-undécane (C11), le n-dodécane (C12), le n-tridécane (C13), le n-tétradecane (C14), et leurs mélanges. Selon un mode de réalisation particulier, l'alcane linéaire volatil est choisi parmi le n-nonane, le n-undécane, le n-dodécane, le n-tridécane, le n-tétradécane, et leurs mélanges.

Selon un mode de réalisation préféré, on peut citer les mélanges de n-undécane (C11) et de n-tridécane (C13) obtenus notamment aux exemples 1 et 2 de la demande WO2008/155059.

On peut également citer le n-dodécane (C12) et le n-tétradécane (C 14) vendus respectivement sous les références PARAFOL 12-97 et PARAFOL 14-97 par la société Sasol, ainsi que leurs mélanges.

Un mode de réalisation consiste à utiliser un seul alcane linéaire volatil.

Alternativement, on peut utiliser un mélange d'au moins deux alcanes linéaires volatils distincts, différant entre eux d'un nombre de carbone n d'au moins 1, en particulier différant entre eux d'un nombre de carbone de 1 ou de 2.

Selon un mode de réalisation, on utilise un mélange d'au moins deux alcanes linéaires volatils distincts comportant de 10 à 14 atomes de carbone et différant entre eux d'un nombre de carbone d'au moins 1. A titre d'exemples, on peut citer notamment les mélanges d'alcanes linéaires volatils C10/C11, C11/C12, ou C12/C13.

Selon un autre mode de réalisation, on utilise un mélange d'au moins deux alcanes linéaires volatils distincts comportant de 10 à 14 atomes de carbone et différant entre eux d'un nombre de carbone d'au moins 2. A titre d'exemples, on peut citer notamment les mélanges d'alcanes linéaires volatils C10/C12, ou C12/C14, pour un nombre de carbone n pair et le mélange C11/C13 pour un nombre de carbone n impair.

Selon un mode de réalisation préféré, on utilise un mélange d'au moins deux alcanes linéaires volatils comportant de 10 à 14 atomes de carbone distincts et différant entre eux d'un nombre de carbone d'au moins 2, et en particulier un mélange d'alcanes linéaires volatils C11/C13 ou un mélange d'alcanes linéaires volatils C12/C14.

D'autres mélanges associant plus de 2 alcanes linéaires volatils selon l'invention, tels que, par exemple, un mélange d'au moins 3 alcanes linéaires volatils comportant de 7 à 15 atomes de carbone distincts et différant entre eux d'un nombre de carbone d'au moins 1, peuvent être utilisés dans l'invention.

Dans le cas des mélanges de deux alcanes linéaires volatils, lesdits deux alcanes linéaires volatils représentent de préférence plus de 95%, et mieux plus de 99% en poids du mélange.

Selon un mode particulier de l'invention, dans un mélange d'alcanes linéaires volatils, l'alcane linéaire volatil ayant le nombre de carbone le plus petit est majoritaire dans le mélange.

Selon un autre mode de l'invention, on utilise un mélange d'alcanes linéaires volatils dans lequel l'alcane linéaire volatil ayant le nombre de carbone le plus grand est majoritaire dans le mélange.

A titre d'exemples de mélanges convenant à l'invention, on peut citer notamment les mélanges suivants :
de 50 à 90% en poids, de préférence de 55 à 80% en poids, préférentiellement encore de 60 à 75% en poids d'alcane linéaire volatil en Cₙ avec n allant de 7 à 15,
de 10 à 50% en poids, de préférence de 20 à 45% en poids, de préférence de 24 à 40% en poids, d'alcane linéaire volatil en Cₙ₊ₓ avec x supérieur ou égal à 1, de préférence x=1 ou x=2, avec n+x compris entre 8 et 14,
par rapport au poids total des alcanes dans ledit mélange.

En particulier, ledit mélange d'alcanes linéaires volatils peut contenir en outre :
moins de 2% en poids, de préférence moins de 1% en poids d'hydrocarbures ramifiés,
et/ou moins de 2% en poids, de préférence moins de 1% en poids d'hydrocarbures aromatiques,
et/ou moins de 2% en poids, de préférence moins de 1% en poids et préférentiellement moins de 0,1% en poids d'hydrocarbures insaturés,
lesdits pourcentages étant exprimés par rapport au poids total du mélange.

Plus particulièrement, les alcanes linéaires volatils convenant dans l'invention peuvent être mis en oeuvre sous la forme d'un mélange n-undécane/n-tridécane.

En particulier, on utilisera un mélange d'alcanes linéaires volatils comprenant :
de 55 à 80% en poids, de préférence de 60 à 75% en poids d'alcane linéaire volatil en C11 (n-undécane) et
de 20 à 45% en poids, de préférence de 24 à 40% en poids d'alcane linéaire volatil en C13 (n-tridécane),
par rapport au poids total des alcanes dans ledit mélange.

Selon un mode de réalisation particulier, le mélange d'alcanes est un mélange n-undécane/n-tridécane. En particulier, un tel mélange peut être obtenu selon l'exemple 1 ou l'exemple 2 de la demande WO 2008/155059.

Selon un autre mode de réalisation particulier, on utilise le n-dodécane vendu sous la référence PARAFOL 12-97 par SASOL.

Selon un autre mode de réalisation particulier, on utilise le n-tétradécane vendu sous la référence PARAFOL 14-97 par SASOL.

Selon encore un autre mode de réalisation, on utilise un mélange de n-dodécane et de n-tétradécane, de préférence dans un rapport 85/15 tel que le mélange commercialisé sous la dénomination VEGELIGHT 1214 par la société Biosynthis.

La composition de l'invention comprend de préférence de 2,5% à 90% en poids, plus préférentiellement de 2,6% à 50% en poids d'alcane(s) linéaire(s) volatil(s), en particulier de 3% à 40% en poids, plus particulièrement de 3 à 30% en poids, et mieux encore de 3 à 10% en poids d'alcane(s) linéaire(s) volatil(s), par rapport au poids total de la composition.

La composition selon l'invention comprend un ou plusieurs tensioactifs cationiques.

On entend par « tensioactif cationique », un tensioactif chargé positivement lorsqu'il est contenu dans la composition selon l'invention. Ce tensioactif peut porter une ou plusieurs charges permanentes positives ou contenir une ou plusieurs fonctions cationisables au sein de la composition selon l'invention.

A titre d'exemple de tensioactifs cationiques utilisables dans la composition cosmétique, on peut notamment citer les amines grasses primaires, secondaires ou tertiaires, éventuellement polyoxyalkylénées, ou leurs sels, les sels d'ammonium quaternaire, et leurs mélanges.

Les amines grasses comprennent en général au moins une chaîne hydrocarbonée en C₈-C₃₀. Parmi les amines grasses utilisables selon l'invention, on peut citer par exemple la stéaryl amidopropyl diméthylamine.

A titre de sels d'ammonium quaternaires, on peut notamment citer, par exemple :
- ceux qui présentent la formule générale (I) suivante : 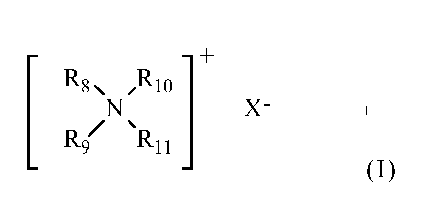
dans laquelle les radicaux R₈ à R₁₁, qui peuvent être identiques ou différents, représentent un radical aliphatique, linéaire ou ramifié, comportant de 1 à 30 atomes de carbone, ou un radical aromatique tel que aryle ou alkylaryle, au moins un des radicaux R₈ à R₁₁ comportant de 8 à 30 atomes de carbones, de préférence de 12 à 24 atomes de carbone. Les radicaux aliphatiques peuvent comporter des hétéroatomes tels que notamment l'oxygène, l'azote, le soufre et les halogènes.

Les radicaux aliphatiques sont par exemple choisis parmi les radicaux alkyle, alcoxy, polyoxyalkylène (C₂-C₆), alkylamide, alkyl(C₁₂-C₂₂)amidoalkyle(C₂-C₆), alkyl(C₁₂-C₂₂)acétate, hydroxyalkyle, comportant environ de 1 à 30 atomes de carbone ; X⁻est un anion choisi dans le groupe des halogénures, phosphates, acétates, lactates, alkyl(C₂-C₆)sulfates, alkyl- ou alkylaryl-sulfonates ;
- les sels d'ammonium quaternaire de l'imidazoline, comme par exemple ceux de formule (II) suivante : 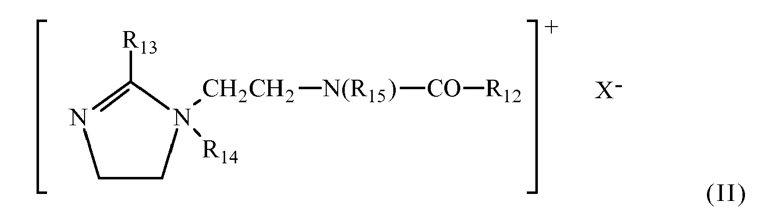
dans laquelle R₁₂ représente un radical alcényle ou alkyle comportant de 8 à 30 atomes de carbone, par exemple dérivés des acides gras du suif, R₁₃ représente un atome d'hydrogène, un radical alkyle en C₁-C₄ ou un radical alcényle ou alkyle comportant de 8 à 30 atomes de carbone, R₁₄ représente un radical alkyle en C₁-C₄, R₁₅ représente un atome d'hydrogène, un radical alkyle en C₁-C₄, X- est un anion choisi dans le groupe des halogénures, phosphates, acétates, lactates, alkylsulfates, alkyl- ou alkylaryl-sulfonates. De préférence, R₁₂ et R₁₃ désignent un mélange de radicaux alcényle ou alkyle comportant de 12 à 21 atomes de carbone, par exemple dérivés des acides gras du suif, R₁₄ désigne un radical méthyle, R₁₅ désigne un atome d'hydrogène. Un tel produit est par exemple commercialisé sous la dénomination REWOQUAT^{®} W 75 par la société REWO ;
- les sels de diammonium quaternaire de formule (III) : 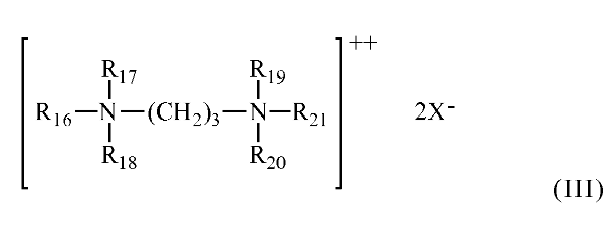
dans laquelle R₁₆ désigne un radical aliphatique comportant environ de 16 à 30 atomes de carbone, R₁₇, R₁₈, R₁₉, R₂₀ et R₂₁, identiques ou différents, sont choisis parmi l'hydrogène ou un radical alkyle comportant de 1 à 4 atomes de carbone, et X⁻ est un anion choisi dans le groupe des halogénures, acétates, phosphates, nitrates et méthylsulfates. De tels sels de diammonium quaternaire comprennent notamment le dichlorure de propane suif diammonium ;
- les sels d'ammonium quaternaire contenant au moins une fonction ester, tels que ceux de formule (IV) suivante : 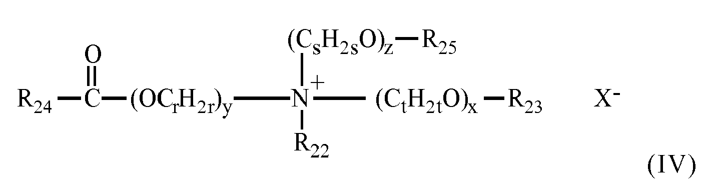
dans laquelle :
R₂₂ est choisi parmi les radicaux alkyles en C₁-C₆ et les radicaux hydroxyalkyles ou dihydroxyalkyles en C₁-C₆ ;
R₂₃ est choisi parmi :
   - le radical 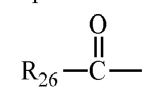
   - les radicaux R₂₇ qui sont des radicaux hydrocarbonés en C₁-C₂₂, linéaires ou ramifiés, saturés ou insaturés,
   - l'atome d'hydrogène,
R₂₅ est choisi parmi :
   - le radical 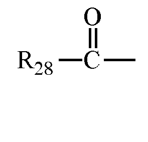
   - les radicaux R₂₉ qui sont des radicaux hydrocarbonés en C₁-C₆, linéaires ou ramifiés, saturés ou insaturés,
   - l'atome d'hydrogène,
R₂₄, R₂₆ et R₂₈, identiques ou différents, sont choisis parmi les radicaux hydrocarbonés en C₇-C₂₁, linéaires ou ramifiés, saturés ou insaturés ;
r, s et t, identiques ou différents, sont des entiers valant de 2 à 6 ;
y est un entier valant de 1 à 10 ;
x et z, identiques ou différents, sont des entiers valant de 0 à 10 ;
X⁻ est un anion simple ou complexe, organique ou inorganique ; sous réserve que la somme x + y + z vaut de 1 à 15, que lorsque x vaut 0 alors R₂₃ désigne R₂₇ et que lorsque z vaut 0 alors R₂₅ désigne R₂₉.

Les radicaux alkyles R₂₂ peuvent être linéaires ou ramifiés et plus particulièrement linéaires.

De préférence R₂₂ désigne un radical méthyle, éthyle, hydroxyéthyle ou dihydroxypropyle, et plus particulièrement un radical méthyle ou éthyle.

Avantageusement, la somme x + y + z vaut de 1 à 10.

Lorsque R₂₃ est un radical R₂₇ hydrocarboné, il peut être long et avoir de 12 à 22 atomes de carbone, ou court et avoir de 1 à 3 atomes de carbone.

Lorsque R₂₅ est un radical R₂₉ hydrocarboné, il a de préférence 1 à 3 atomes de carbone.

Avantageusement, R₂₄, R₂₆ et R₂₈, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1. Avantageusement, y est égal à 1.

De préférence, r, s et t, identiques ou différents, valent 2 ou 3, et encore plus particulièrement sont égaux à 2.

L'anion X- est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X⁻ est encore plus particulièrement le chlorure ou le méthylsulfate.

On utilise plus particulièrement dans la composition selon l'invention, les sels d'ammonium de formule (IV) dans laquelle :
- R₂₂ désigne un radical méthyle ou éthyle,
- x et y sont égaux à 1 ;
- z est égal à 0 ou 1 ;
- r, s et t sont égaux à 2 ;
- R₂₃ est choisi parmi :
- le radical 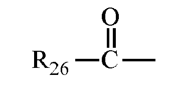
- les radicaux méthyle, éthyle ou hydrocarbonés en C₁₄-C₂₂,
- l'atome d'hydrogène ;
- R₂₅ est choisi parmi :
- le radical 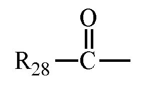
- l'atome d'hydrogène ;
- R₂₄, R₂₆ et R₂₈, identiques ou différents, sont choisis parmi les radicaux hydrocarbonés en C₁₃-C₁₇, linéaires ou ramifiés, saturés ou insaturés, et de préférence parmi les radicaux alkyles et alcényles en C₁₃-C₁₇, linéaires ou ramifiés, saturés ou insaturés.

Avantageusement, les radicaux hydrocarbonés sont linéaires. On peut citer par exemple les composés de formule (IV) tels que les sels (chlorure ou méthylsulfate notamment) de diacyloxyéthyl-diméthylammonium, de diacyloxyéthyl-hydroxyéthyl-méthylammonium, de monoacyloxyéthyl-dihydroxyéthyl-méthylammonium, de triacyloxy éthyl-méthylammonium, de monoacyloxyéthyl-hydroxyéthyl-diméthyl ammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthane sulfonate de méthyle, le paratoluènesulfonate de méthyle, la chlorhydrine du glycol ou du glycérol.

De tels composés sont par exemple commercialisés sous les dénominations DEHYQUART^{®} par la société HENKEL, STEPANQUAT^{®} par la société STEPAN, NOXAMIUM^{®} par la société CECA, REWOQUAT^{®} WE 18 par la société REWO-WITCO.

La composition selon l'invention contient de préférence un mélange de sels de mono-, di- et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl-dihydroxyéthyl-méthylammonium, 45 à 60% de méthyl sulfate de diacyloxyéthyl-hydroxyéthyl-méthylammonium et 15 à 30% de méthylsulfate de triacyloxyéthyl-méthylammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

Parmi les sels d'ammonium quaternaire de formule (I), on préfère d'une part, les chlorures de tétraalkylammonium comme, par exemple, les chlorures de dialkyldiméthylammonium ou d'alkyltriméthylammonium dans lesquels le radical alkyle comporte environ de 12 à 22 atomes de carbone, en particulier les chlorures de béhényltriméthylammonium, de distéaryldiméthylammonium, de cétyltriméthylammonium, de benzyldiméthylstéarylammonium ou encore, d'autre part, le méthosulfate de distéaroyléthyl hydroxyéthyl méthylammonium, le méthosulfate de dipalmitoyléthylhydroxyéthylammonium ou le méthosulfate de distéaroyléthyl hydroxyéthylammonium, ou encore, enfin, le chlorure de palmitylamidopropyltriméthylammonium ou le chlorure de stéaramidopropyldiméthyl-(myristyl acétate)-ammonium commercialisé sous la dénomination CERAPHYL^{®} 70 par la société VAN DYK.

Parmi tous les tensioactifs cationiques pouvant être présents dans la composition selon l'invention, on préfère choisir le ou les tensioactifs cationiques parmi les sels de (chlorure ou méthosulfate) de cétyltriméthylammonium (INCI cetrimonium-), de béhényltriméthylammonium (INCI behentrimonium-), de dipalmitoyléthylhydroxyéthylmethylammonium, de distéaroyléthylhydroxyéthyl méthylammonium, de méthyl alkyl(C₉-C₁₉) alkyl(C₁₀-C₂₀)amidoéthylimidazolium, la stéaramidopropyldiméthylamine, le sel de stéaramidopropyldiméthylammonium, et leurs mélanges.

La composition de l'invention comprend de préférence de 0,1 à 10% en poids, en particulier de 0,5 à 8% en poids, et mieux encore de 1 à 5% en poids de tensioactifs cationiques, par rapport au poids total de la composition.

La composition selon l'invention peut comprendre en outre au moins une silicone.

La ou les silicones éventuellement présentes dans la composition selon l'invention sont en particulier des polyorganosiloxanes qui peuvent se présenter sous forme de solutions aqueuses, c'est-à-dire solubilisées, ou éventuellement sous forme de dispersions ou micro-dispersions, ou d'émulsions aqueuses. Les polyorganosiloxanes peuvent également se présenter sous forme d'huiles, de cires, de résines ou de gommes.

Les organopolysiloxanes sont définis plus en détail dans l'ouvrage de Walter NOLL "Chemistry and Technology of Silicones" (1968) Academie Press.

La ou les silicones peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et de préférence 4 à 5.

Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHONE POULENC, le décaméthylcyclopentasiloxane commercialisé sous le nom de "VOLATILE SILICONE 7158" par UNION CARBIDE, "SILBIONE 70045 V 5" par RHONE POULENC, ainsi que leurs mélanges.

On peut également citer les cyclocopolymères du type diméthylsiloxanes/méthylalkylsiloxane, tel que la "SILICONE VOLATILE FZ 3109" commercialisée par la société UNION CARBIDE, de structure chimique :

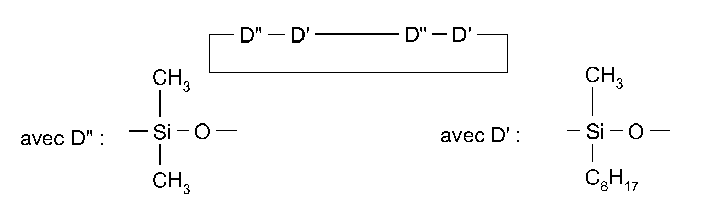

On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25°C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle (Diméthicone selon la dénomination CTFA) ayant une viscosité de 5.10⁻⁶ à 2,5 m²/s à 25°C et de préférence 1.10⁻⁵ à 1 m²/s. La viscosité des silicones est par exemple mesurée à 25°C selon la norme ASTM 445 Appendice C.

Parmi ces polyalkylsiloxanes, on peut citer à titre non limitatif les produits commerciaux suivants :
- les huiles SILBIONE des séries 47 et 70 047 ou les huiles MIRASIL commercialisées par RHONE POULENC telles que par exemple l'huile 70 047 V 500 000 ;
- les huiles de la série MIRASIL commercialisées par la société RHONE POULENC ;
- les huiles de la série 200 de la société DOW CORNING telles que plus particulièrement la DC200 de viscosité 60 000 cSt (centistokes);
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHONE POULENC.

On peut également citer les polydiméthylsiloxanes à groupements aminoéthyl aminopropyl et alpha-oméga silanols.

Dans cette classe de polyalkylsiloxanes, on peut également citer les produits commercialisés sous les dénominations "ABIL WAX 9800 et 9801" par la société GOLDSCHMIDT qui sont des polyalkyl (C₁-C₂₀) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de 1.10⁻⁵ à 5.10⁻²m²/s à 25°C.

Parmi ces polyalkylarylsiloxanes on peut citer à titre d'exemple les produits commercialisés sous les dénominations suivantes :
- les huiles SILBIONE de la série 70 641 de RHONE POULENC ;
- les huiles des séries RHODORSIL 70 633 et 763 de RHONE POULENC ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Les gommes de silicone pouvant être présentes dans la composition selon l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- les gommes de polydiméthylsiloxane,
- les gommes polydiméthylsiloxanes/méthylvinylsiloxane,
- les gommes poly[(diméthylsiloxane)/(vinylhydrogénosiloxane),
- les gommes poly[(diméthylsiloxane)/(divinyldihydrogéno-siloxane),
- les gommes de polydiméthylsiloxane/diphénylsiloxane,
- les gommes de polydiméthylsiloxane/phénylméthylsiloxane,
- les gommes de polydiméthylsiloxane / diphénylsiloxane/méthylvinylsiloxane.

Des produits plus particulièrement utilisables sont les mélanges suivants:
- les mélanges formés à partir d'un polydiméthylsiloxane hydroxylé en bout de chaîne (dénommé diméthiconol selon la nomenclature du dictionnaire CTFA) et d'un poly-diméthylsiloxane cyclique (dénommé cyclométhicone selon la nomenclature du dictionnaire CTFA) tel que le produit Q2 1401 commercialisé par la société DOW CORNING ;
- les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15% en poids de gomme SE 30 et 85 % en poids d'une huile SF 96.

Les résines d'organopolysiloxanes éventuellement présentes dans la composition selon l'invention sont des systèmes siloxaniques réticulés renfermant les unités : R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées éventuellement présentes dans la composition selon l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que le poly méthyllauryl/méthylsiloxane oxyéthyléné et oxypropyléné commercialisé sous le nom Dow Corning 5200 formulation aid par la société Dow Corning (INCI : Lauryl PEG/PPG-18/18 methicone), les produits dénommés diméthicone copolyol commercialisé par la société DOW CORNING sous la dénomination DC 1248, les huiles SILWET L 722, L 7500, L 77, L 711 de la société UNION CARBIDE) ou l'alkyl (C₁₂) méthicone copolyol commercialisée par la société DOW CORNING sous la dénomination Q2 5200 ;
- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;
- des groupements hydroxylés comme les polyorganosiloxanes à fonction hydroxyalkyle décrits dans la demande de brevet français FR-A-85 16334 ;
- des groupements acyloxyalkyle tels que par exemple les polyorganosiloxanes décrits dans le brevet US-A-4957732 ;
- des groupements anioniques du type phosphate ou carboxylique comme par exemple dans les produits décrits dans le brevet EP 186 507 de la société CHISSO CORPORATION, ou de type alkylcarboxyliques comme ceux présents dans le produit X-22-3701E de la société SHIN-ETSU ; 2-hydroxyalkylsulfonate 2-hydroxyalkylthiosulfate tels que les produits commercialisés par la société GOLDSCHMIDT sous les dénominations "ABIL S201" et "ABIL S255" ;
- des groupements hydroxyacylamino, comme les polyorganosiloxanes décrits dans la demande EP 342 834. On peut citer par exemple le produit Q2-8413 de la société DOW CORNING.

Parmi les silicones organomodifiées, on peut encore citer les silicones aminées.

Par « silicone aminée », on désigne tout polyaminosiloxane, c'est-à-dire tout polysiloxane comportant au moins une fonction amine primaire, secondaire, tertiaire ou un groupement ammonium quaternaire.

De préférence, la ou les silicones aminées utilisées dans la composition cosmétique selon la présente invention sont choisies parmi :
(a) les composés répondant à la formule (V) suivante :

   (R¹)ₐ(T)₃₋ₐ-Si[OSi(T)₂]ₙ-[OSi(T)_{b}(R¹)_{2-b}]ₘ-OSi(T)₃₋ₐ-(R¹)ₐ (V)

   dans laquelle :
   T est un atome d'hydrogène, ou un radical phényle, hydroxyle (-OH), ou alkyle en C₁-C₈, et de préférence méthyle ou alcoxy en C₁-C₈, de préférence méthoxy,
   a désigne le nombre 0 ou un nombre entier de 1 à 3, et de préférence 0,
   b désigne 0 ou 1, et en particulier 1,
   m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
   R¹ est un radical monovalent de formule -C_{q}H_{2q}L dans laquelle q est un nombre de 2 à 8 et L est un groupement aminé éventuellement quaternisé choisi parmi les groupements :
      - N(R²)-CH₂-CH₂-N(R²)₂ ;
      - N(R²)₂ ; -N⁺(R² )₃ Q ;
      - N⁺(R²) (H)₂ Q⁻ ;
      - N⁺(R²)₂HQ⁻ ;
      - N(R²)-CH₂-CH₂-N⁺(R²)(H)₂ Q⁻,
   dans lesquels R² désigne un atome d'hydrogène, un phényle, un benzyle, ou un radical hydrocarboné saturé monovalent, par exemple un radical alkyle en C₁-C₂₀, et Q- représente un ion halogénure tel que par exemple fluorure, chlorure, bromure ou iodure.

En particulier, les silicones aminées correspondant à la définition de la formule (V) sont choisies parmi les composés correspondant à la formule (VI) suivante :

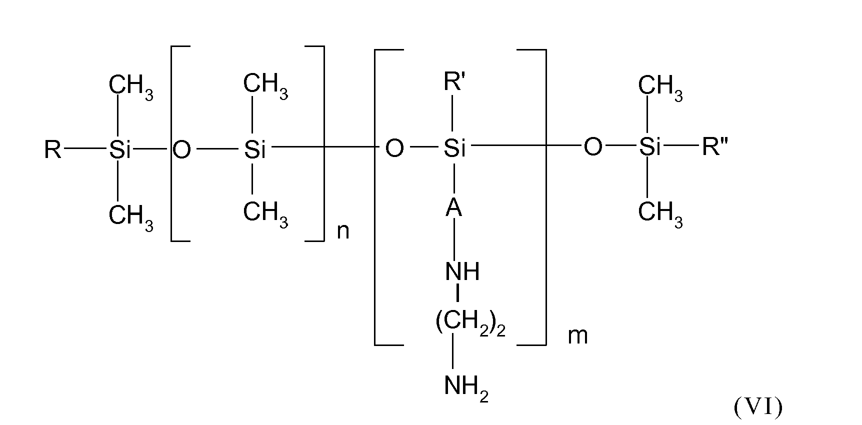

dans laquelle R, R', R", identiques ou différents, désignent un radical alkyle en C₁-C₄, de préférence CH₃ ; un radical alcoxy en C₁-C₄, de préférence méthoxy ; ou OH ; A représente un radical alkylène, linéaire ou ramifié, en C₃-C₈, de préférence en C₃-C₆; m et n sont des nombres entiers dépendant du poids moléculaire et dont la somme est comprise entre 1 et 2000.

Selon une première possibilité, R, R', R", identiques ou différents, représentent un radical alkyle en C₁-C₄ ou hydroxyle, A représente un radical alkylène en C₃ et m et n sont tels que la masse moléculaire moyenne en poids du composé est comprise entre 5 000 et 500 000 environ. Des composés de ce type sont dénommés dans le dictionnaire CTFA, "amodiméthicone"

Selon une deuxième possibilité, R, R', R", identiques ou différents, représentent un radical alcoxy en C₁-C₄ ou hydroxyle, l'un au moins des radicaux R ou R" est un radical alcoxy et A représente un radical alkylène en C₃. Le rapport molaire hydroxy / alcoxy est de préférence compris entre 0,2/1 et 0,4/1 et avantageusement égal à 0,3/1. Par ailleurs, m et n sont tels que la masse moléculaire moyenne en poids du composé est comprise entre 2000 et 10⁶. Plus particulièrement, n est compris entre 0 et 999 et m est compris entre 1 et 1000, la somme de n et m étant comprise entre 1 et 1000.

Dans cette catégorie de composés, on peut citer entre autres, le produit Belsil®ADM 652, commercialisée par Wacker.

Selon une troisième possibilité, R, R", différents, représentent un radical alcoxy en C₁-C₄ ou hydroxyle, l'un au moins des radicaux R, R" est un radical alcoxy, R' représente un radical méthyle et A représente un radical alkylène en C₃. Le rapport molaire hydroxy / alcoxy est de préférence compris entre 1/0,8 et 1/1,1, et avantageusement est égal à 1/0,95. Par ailleurs, m et n sont tels que la masse moléculaire moyenne en poids du composé est comprise entre 2000 et 200000. Plus particulièrement, n est compris entre 0 et 999 et m est compris entre 1 et 1000, la somme de n et m étant comprise entre 1 et 1000.

Plus particulièrement, on peut citer le produit FluidWR® 1300, commercialisé par Wacker.

Selon une quatrième possibilité, R, R" représentent un radical hydroxyle, R' représente un radical méthyle et A est un radical alkylène, en C₄-C₈, de préférence en C₄. Par ailleurs, m et n sont tels que la masse moléculaire moyenne en poids du composé est comprise entre 2000 et 10⁶. Plus particulièrement, n est compris entre 0 et 1999 et m est compris entre 1 et 2000, la somme de n et m étant comprise entre 1 et 2000.

Un produit de ce type est notamment commercialisé sous la dénomination DC28299 par Dow Corning.

Notons que la masse moléculaire de ces silicones est déterminée par chromatographie par perméation de gel (température ambiante, étalon polystyrène ; colonnes µ styragem ; éluant THF ; débit de 1 mm/m ; on injecte 200 µl d'une solution à 0,5 % en poids de silicone dans le THF et l'on effectue la détection par réfractométrie et UV-métrie).

Un produit correspondant à la définition de la formule (V) est en particulier le polymère dénommé dans le dictionnaire CTFA (7^{ème} édition 1997) "triméthylsilylamodiméthicone", répondant à la formule (VII) suivante:

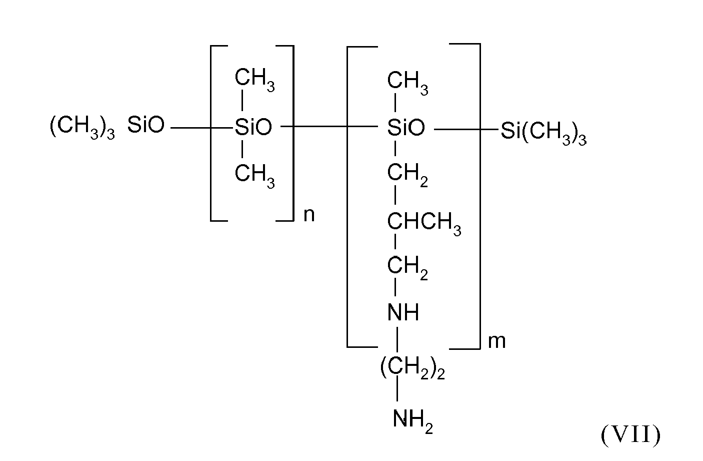

dans laquelle n et m ont les significations données ci-dessus conformément à la formule (V) ou (VI).

De tels composés sont décrits par exemple dans EP 0095238; un composé de formule (VII) est par exemple vendu sous la dénomination Q2-8220 par la société OSI.
(b) les composés répondant à la formule (VIII) suivante : 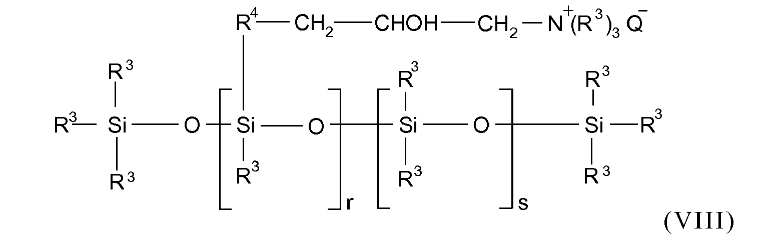 dans laquelle :
   R³ représente un radical hydrocarboné monovalent en C₁-C₁₈, et en particulier un radical alkyle en C₁-C₁₈ ou alcényle en C₂-C₁₈, par exemple méthyle ;
   R⁴ représente un radical hydrocarboné divalent, notamment un radical alkylène en C₁-C₁₈ ou un radical alkylèneoxy divalent en C₁-C₁₈, par exemple en C₁-C₈;
   Q⁻ est un ion halogénure, notamment chlorure ;
   r représente une valeur statistique moyenne de 2 à 20 et en particulier de 2 à 8 ;
   s représente une valeur statistique moyenne de 20 à 200 et en particulier de 20 à 50.

De tels composés sont décrits plus particulièrement dans le brevet US 4185087.

Un composé entrant dans cette classe est celui vendu par la Société Union Carbide sous la dénomination "Ucar Silicone ALE 56".
(c) les silicones ammonium quaternaire de formule (IX) : 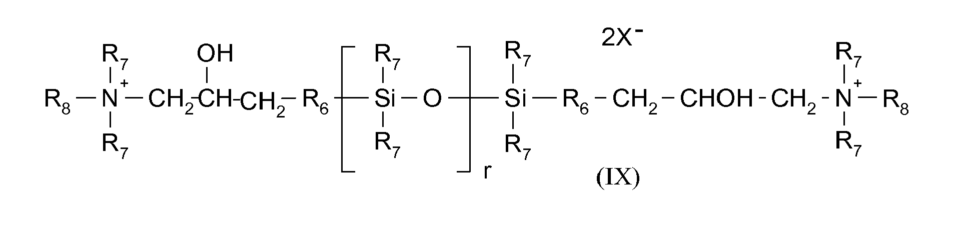 dans laquelle :
   R₇, identiques ou différents, représentent un radical hydrocarboné monovalent ayant de 1 à 18 atomes de carbone, et en particulier un radical alkyle en C₁-C₁₈, un radical alcényle en C₂-C₁₈ ou un cycle comprenant 5 ou 6 atomes de carbone, par exemple méthyle ;
   R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC;
   R₈, identiques ou différents, représentent un atome d'hydrogène, un radical hydrocarboné monovalent ayant de 1 à 18 atomes de carbone, et en particulier un radical alkyle en C₁-C₁₈, un radical alcényle enC₂-C₁₈ , un radical -R₆-NHCOR₇ ,
   X⁻ est un anion tel qu'un ion halogénure, notamment chlorure ou un sel d'acide organique (acétate ...);
   r représente une valeur statistique moyenne de 2 à 200 et en particulier de 5 à 100 ;
   Ces silicones sont par exemple décrites dans la demande EP-A-0530974.
d) les silicones aminées de formule suivante : 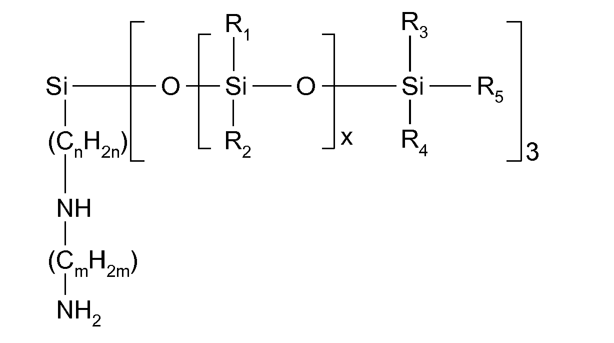 dans laquelle :
   - R₁, R₂, R₃ et R₄, identiques ou différents, désignent un radical alkyle en C₁-C₄ ou un groupement phényle,
   - R₅ désigne un radical alkyle en C₁-C₄ ou un groupement hydroxyle,
   - n est un entier variant de 1 à 5,
   - m est un entier variant de 1 à 5,
   et dans laquelle x est choisi de manière telle que l'indice d'amine soit compris entre 0,01 et 1 méq/g.

La ou les silicones particulièrement préférées sont les polysiloxanes à groupements aminés tels que les silicones de formule (VI) ou les silicones de formule (VII), et encore plus particulièrement les silicones à groupements ammonium quaternaire tels que les silicones de formule (IX).

Lorsque ces composés sont mis en oeuvre, une forme de réalisation particulièrement intéressante est leur utilisation conjointe avec des agents de surface cationiques et/ou non ioniques.

A titre exemple, on peut utiliser le produit vendu sous la dénomination "Emulsion Cationique DC 929" par la Société Dow Corning, qui comprend, outre l'amodiméthicone, un agent de surface cationique comprenant un mélange de produits répondant à la formule (X) : 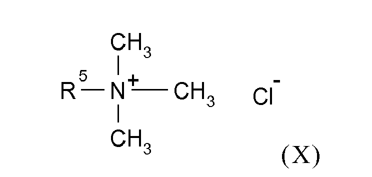 dans laquelle, R⁵ désigne des radicaux alcényle et/ou alcoyle en C₁₄-C₂₂ dérivés des acides gras du suif, et connu sous la dénomination CTFA "tallowtrimonium chloride",en association avec un agent de surface non ionique de formule :
C₉H₁₉-C₆H₄-(OC₂H₄)₁₀-OH, connu sous la dénomination CTFA "Nonoxynol 10".

On peut également utiliser par exemple le produit vendu sous la dénomination "Emulsion Cationique DC 939" par la Société Dow Corning, qui comprend, outre l'amodiméthicone, un agent de surface cationique qui est le chlorure de triméthylcétylammonium et un agent de surface non ionique de formule : C₁₃H₂₇-(OC₂H₄)₁₂-OH, connu sous la dénomination CTFA "tridéceth-12".

Un autre produit commercial utilisable selon l'invention est le produit vendu sous la dénomination "Dow Corning Q2 7224" par la Société Dow Corning, comportant en association le triméthylsilylamodiméthicone de formule (C) décrite ci-dessus, un agent de surface non ionique de formule : C₈H₁₇-C₆H₄-(OCH₂CH₂)₄₀-OH, connu sous la dénomination CTFA "octoxynol-40", un second agent de surface non ionique de formule: C₁₂H₂₅-(OCH₂-CH₂)₆-OH, connu sous la dénomination CTFA "isolaureth-6", et du propylèneglycol.

Parmi toutes les silicones pouvant être présentes dans la composition selon l'invention, on préfère choisir la ou les silicones parmi les silicones non volatiles et plus particulièrement les polyalkylsiloxanes, les polyarylsiloxanes, les polyalkylarylsiloxanes, les gommes de silicones, les polyorganosiloxanes modifiés par des groupements organofonctionnels choisies parmi les silicones aminées et les silicones comportant des groupements polyéthylèneoxy et/ou polypropylèneoxy, ainsi que leurs mélanges.

De manière plus préférée, la ou les silicones sont choisies parmi les poly diméthyl siloxanes éventuellement sous forme de gommes, les gommes poly[(diméthylsiloxane) / (vinylhydrogénosiloxane)], les polyorganosiloxanes modifiés par des groupements organofonctionnels et choisies parmi les silicones aminées ou les silicones comportant des groupements polyéthylèneoxy et/ou polypropylèneoxy, ainsi que leurs mélanges.

Lorsque la composition selon l'invention comprend au moins une silicone, la ou les silicones représentent généralement de 0,5 à 20%, de préférence de 0,1 à 10% en poids du poids total de la composition.

La composition selon l'invention peut également comprendre un ou plusieurs alcools gras en C₈-C₄₀.

Les alcools gras en C₈-C₄₀ peuvent être choisis parmi les alcools de formule R'OH, où R' désigne un radical saturé ou insaturé, linéaire ou ramifié, comportant de 8 à 40 atomes de carbone, de préférence 8 à 30 atomes de carbone. R' désigne de préférence un groupement alkyle en C₁₂-C₂₄ ou alcényle en C₁₂-C₂₄. R' peut être substitué par un ou plusieurs groupements hydroxy.

Le ou les alcools gras peuvent être en particulier choisis parmi l'alcool laurique, l'alcool cétylique, l'alcool dodécylique, l'alcool décylique, l'alcool stéarylique, l'alcool oléïque, l'alcool béhénique, l'alcool linoléique, l'alcool undécylénique, l'alcool palmitoléïque, l'alcool arachidonique, l'alcool myristylique et l'alcool érucique. On peut également utiliser un mélange d'alcools gras, ce qui signifie que dans un produit commercial peuvent coexister plusieurs espèces d'alcools gras, sous forme d'un mélange. A titre de mélange d'alcools gras, on peut citer l'alcool cétylstéarylique ou cétéarylique.

De préférence, le ou les alcools gras sont choisis parmi l'alcool myristique, l'alcool cétylique, l'alcool stéarylique et leurs mélanges.

Lorsque la composition selon l'invention comprend au moins un alcool gras, le ou les alcools gras représentent généralement de 0,5 à 25%, de préférence de 1 à 10% en poids du poids total de la composition.

La composition selon l'invention peut également comprendre un ou plusieurs polymères cationiques.

Par « polymère cationique », on entend au sens de la présente invention, tout polymère comprenant des groupements cationiques et/ou des groupements ionisables en groupements cationiques.

Le ou les polymères cationiques utilisables dans la composition cosmétique selon l'invention sont de préférence choisis parmi les polymères comportant des groupements amine primaire, secondaire, tertiaire et/ou quaternaire faisant partie de la chaîne polymère ou directement reliés à celle-ci, et ayant une masse moléculaire moyenne en nombre comprise entre 500 et environ 5 000 000, et de préférence entre 1 000 et 3 000 000.

Parmi ces polymères, on peut citer plus particulièrement les polymères cationiques suivants :
(1) les homopolymères ou copolymères d'esters ou d'amides acryliques ou méthacryliques, à fonctions aminées, comportant au moins un des motifs de formules suivantes : 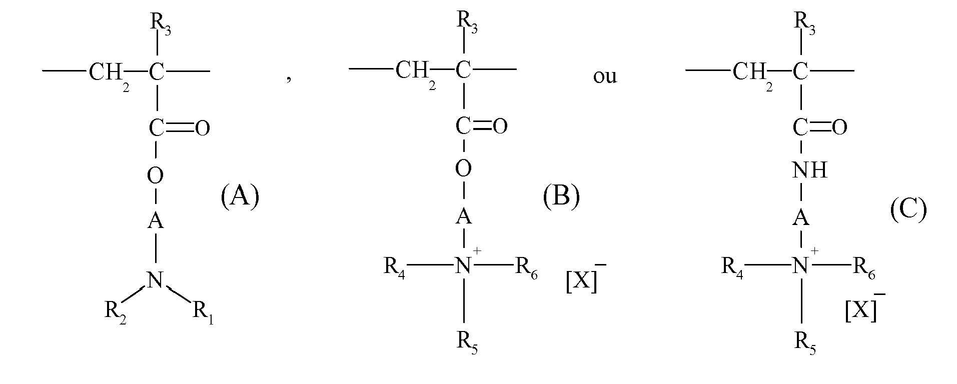 dans lesquelles:
   R₁ et R₂, identiques ou différents, représentent chacun un atome d'hydrogène ou un groupe alkyle ayant de 1 à 6 atomes de carbone ;
   R₃ désigne un atome d'hydrogène ou un groupe CH₃ ;
   A est un groupe alkyle linéaire ou ramifié, comportant de 1 à 6 atomes de carbone ou un groupe hydroxyalkyle comportant de 1 à 4 atomes de carbone ;
   R₄, R₅, R₆, identiques ou différents, représentent un groupe alkyle ayant de 1 à 18 atomes de carbone ou un groupe benzyle ;
   X⁻ désigne un anion méthosulfate ou un halogénure tel que chlorure ou bromure.
   Les copolymères de la famille (1) contiennent en outre un ou plusieurs motifs dérivant de comonomères pouvant être choisis dans la famille des acrylamides, méthacrylamides, diacétone-acrylamides, acrylamides et méthacrylamides substitués sur l'azote par des groupes alkyle inférieur (C₁₋₄), des groupes dérivés des acides acryliques ou méthacryliques ou de leurs esters, de vinyllactames tels que la vinylpyrrolidone ou le vinylcaprolactame, d'esters vinyliques.
   Ainsi, parmi ces copolymères de la famille (1), on peut citer :
   - les copolymères d'acrylamide et de méthacrylate de diméthylaminoéthyle quaternisés au sulfate de diméthyle ou avec un halogénure de diméthyle, tels que celui vendu sous la dénomination HERCOFLOC^{®} par la société HERCULES,
   - les copolymères d'acrylamide et de chlorure de méthacryloyloxyéthyltriméthylammonium décrits, par exemple, dans la demande de brevet EP-A-080976 et vendus sous la dénomination BINA QUAT P 100 par la société CIBA GEIGY,
   - les copolymères d'acrylamide et de méthosulfate de méthacryloyloxyéthyltriméthylammonium, tels que celui vendu sous la dénomination RETEN par la société HERCULES,
   - les copolymères vinylpyrrolidone/acrylate ou méthacrylate de dialkylaminoalkyle quaternisés ou non, tels que les produits vendus sous la dénomination "GAFQUAT^{®}" par la société ISP comme, par exemple, "GAFQUAT^{®} 734" ou "GAFQUAT^{®} 755", ou bien les produits dénommés "COPOLYMER^{®} 845, 958 et 937". Ces polymères sont décrits en détail dans les brevets français Nos 2 077 143 et 2 393 573,
   - les terpolymères méthacrylate de diméthylaminoéthyle/ vinylcaprolactame/vinylpyrrolidone tels que le produit commercialisé sous la dénomination GAFFIX^{®} VC 713 par la société ISP, et
   - les copolymères vinylpyrrolidone/méthacrylamide de diméthylaminopropyle quaternisé tels que notamment le produit commercialisé sous la dénomination "GAFQUAT^{®} HS 100" par la société ISP ;
   - les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE® SC 92 " par la Société CIBA. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE® SC 95 " et " SALCARE® SC 96 " par la Société CIBA.
(2) les polysaccharides cationiques, et en particulier ceux choisis parmi :
   a) les dérivés d'éthers de cellulose comportant des groupements ammonium quaternaire décrits dans le brevet FR 1492597. Ces polymères sont également définis dans le dictionnaire CTFA comme des ammoniums quaternaires d'hydroxyéthyl cellulose ayant réagi avec un époxyde substitué par un groupement triméthylammonium ;
   b) les dérivés de cellulose greffés avec un monomère hydrosoluble comportant un ammonium quaternaire, et décrits notamment dans le brevet US 4 131 576, tels que les hydroxyalkylcelluloses, comme les hydroxyméthyl-, hydroxyéthyl- ou hydroxypropyl-celluloses greffées notamment avec un sel de méthacryloyloxyéthyl-triméthylammonium, de méthacrylamidopropyl triméthylammonium, de diméthyl-diallylammonium.
      Les produits commercialisés répondant à cette définition sont plus particulièrement les produits vendus sous la dénomination "CELQUAT L 200" et "CELQUAT H 100" par la Société National Starch.
   c) les polygalactomananes cationiques tels que ceux décrits dans les brevets américains 3 589 578 et 4 031 307 tel que les gommes de guar contenant des groupements cationiques trialkylammonium. De tels produits sont commercialisés notamment sous les dénominations commerciales de JAGUAR C13 S, JAGUAR C 15, JAGUAR C 17 par la société RHODIA ;
(3) les copolymères quaternaires de vinylpyrrolidone et de vinylimidazole ;
(4) les chitosanes ou leurs sels ; les sels utilisables sont en particulier l'acétate, le lactate, le glutamate, le gluconate ou le pyrrolidone-carboxylate de chitosane.

Parmi ces composés, on peut citer le chitosane ayant un taux de désacétylation de 90,5 % en poids vendu sous la dénomination KYTAN BRUT STANDARD par la société ABER TECHNOLOGIES, le pyrrolidone-carboxylate de chitosane commercialisé sous la dénomination KYTAMER^{®} PC par la société AMERCHOL.

Parmi tous les polymères cationiques pouvant être présents dans la composition selon l'invention, on préfère choisir le ou les polymères cationiques parmi les polymères réticulés de sels de méthacryloyloxyalkyl(C₁-C₄) trialkyl(C₁-C₄)ammonium.

Lorsque la composition comprend au moins un polymère cationique, celui-ci ou ceux-ci sont présents dans une concentration allant de préférence de 0,1 à 10% en poids du poids total de la composition.

La composition selon l'invention comprend un milieu cosmétiquement acceptable.

Le milieu cosmétiquement acceptable est constitué d'eau ou d'un mélange d'eau et d'au moins un solvant cosmétiquement acceptable de préférence choisi parmi les alcools inférieurs en C₁-C₄, tels que l'éthanol, l'isopropanol, le tertio-butanol ou le n-butanol ; les polyols tels que le glycérol, le propylèneglycol et les polyéthylèneglycols ; et leurs mélanges.

La composition selon l'invention peut comprendre en outre un ou plusieurs additifs classiques bien connus dans la technique, différents des composés définis précédemment. A titre d'exemples d'additifs utilisables selon l'invention, on peut citer des polymères associatifs ou non, ioniques ou non-ioniques, des silanes, des tensioactifs anioniques, des tensioactifs amphotères ou zwittérioniques, des tensioactifs non ioniques, des protéines, des vitamines, des agents réducteurs, des plastifiants, des adoucissants, des agents anti-mousse, des agents hydratants, des pigments, des argiles, des charges minérales, des filtres UV, des colloïdes minéraux, des peptisants, des solubilisants, des parfums, des conservateurs, des agents nacrants, des propulseurs, et des épaississants minéraux ou organiques ; ces additifs étant différents des composés définis plus haut.

L'homme de métier veillera à choisir le ou les éventuels additifs et leur quantité de manière à ce qu'ils ne nuisent pas aux propriétés des compositions de la présente invention.

Le ou les additifs sont généralement présents dans la composition selon l'invention en une quantité allant de 0 à 20 % en poids par rapport au poids total de la composition.

Les compositions selon l'invention peuvent se présenter sous la forme de composition de soin rincé ou non-rincé, ces dernières se présentant sous la forme d'une lotion plus ou moins épaissie, d'une crème, d'un gel ou d'une émulsion.

Un autre objet de l'invention est l'utilisation de la composition cosmétique telle que décrite ci-dessus pour le traitement cosmétique des matières kératiniques, de préférence des fibres kératiniques comme les cheveux, et notamment comme produit capillaire rincé.

L'invention est également relative à un procédé de traitement cosmétique des matières kératiniques, de préférence des fibres kératiniques comme les cheveux, qui comprend l'application d'une quantité efficace d'une composition cosmétique telle que décrite ci-dessus, sur lesdites matières, et éventuellement le rinçage de ladite composition après un éventuel temps de pause.

Lorsque l'on applique la composition selon l'invention sous forme d'une lotion ou d'une crème, on la laisse éventuellement pauser sur les cheveux pendant environ 0,5 à 5 minutes, puis on rince éventuellement à l'eau.

Les exemples suivants sont donnés à titre illustratif de la présente invention.

Dans les exemples suivants, toutes les quantités sont indiquées en pour cent en poids par rapport au poids total de la composition, sauf indication contraire.

### EXEMPLES

On a préparé les compositions de soin rincé A, B et C suivantes à partir des ingrédients indiqués dans le tableau ci-dessous.

| | | | |
|---|---|---|---|
| Compositions | A | B | C |
| Mélange majoritairement composé de n-undécane et de n-tridécane pur selon l'exemple 2 de la demande WO 2008/155059 | 5 | 3 | - |
| Mélange de n-dodécane et de n-tétradécane (VEGELIGHT 1214 -Biosynthis) | - | - | 5 |
| Copolymère polydiméthylsiloxane à groupement alpha-oméga vinyle/ alpha-oméga hydrogéno-polysiloxane à 67% en poids de matière active dans une émulsion cationique comprenant 2,2% en poids de chlorure de cétyl triméthyl ammonium (Dow Corning 2 1997 cationic emulsion ― Dow Corning) | 2 (1,34%MA) | 2 (1,34%MA) | 2 (1,34%MA) |
| Alcool myristylique (Nacol 14-98 - Sasol) | 3 | 3 | 3 |
| Alcool cétylique (Nacol 16-98 - Sasol) | 1 | 1 | 1 |
| Homopolymère de chlorure de méthacrylate d'éthyl triméthyl ammonium réticulé, en émulsion inverse à 50% en poids dans une huile minérale (SALCARE® SC 95 - Ciba) | 0,5 (0,25%MA) | 0,5 (0,25%MA) | 0,5 (0,25%MA) |
| Stéaryl amidopropyl diméthylamine (Mackine 301 -Rhodia) | 2,5 | 2,5 | 2,5 |
| Méthosulfate de distéaroyléthyl hydroxyéthyl méthyl ammonium à 75% en poids dans de l'alcool cétylstéarylique (Dehyquart F75 ― Cognis) | 2,5 (1,875%MA) | 2,5 (1,875%MA) | 2,5 (1,875%MA) |
| Lauryl PEG/PPG-18/18 methicone à 72% en poids de matière active (Dow Corning 5200 formulation aid - Dow Corning) | 1 (0,72%MA) | 1 (0,72%MA) | 1 (0,72%MA) |
| Acide citrique, 1 H₂O | 0,85 | 0,85 | 0,85 |
| Conservateur, Parfum | qs | qs | qs |
| Eau désionisée | Qs 100 | Qs 100 | Qs 100 |
| Rapport pondéral de la quantité d'alcanes linéaires volatils sur la quantité de tensioactifs cationiques | 1,13 | 0,68 | 1,13 |

| | | | |
|---|---|---|---|
| %MA : pourcentage en poids de Matière Active par rapport au poids total de la composition | | | |

On a observé que les compositions selon l'invention A, B et C conduisent à un bon lissage de la fibre capillaire lors du rinçage et sur cheveux séchés.

## Revendications

1. Composition cosmétique comprenant dans un milieu cosmétiquement acceptable :
- au moins 2,5% en poids d'un ou plusieurs alcanes linéaires volatils par rapport au poids total de la composition, et
- un ou plusieurs tensioactifs cationiques,
le rapport pondéral de la quantité d'alcane(s) linéaire(s) volatil(s) sur la quantité de tensioactif(s) cationique(s) étant inférieur ou égal à 1,5.

2. Composition cosmétique selon la revendication précédente, **caractérisée en ce que** le ou les alcanes linéaires volatils sont les alcanes linéaires comportant de 7 à 15 atomes de carbone.

3. Composition cosmétique selon l'une quelconque des revendications précédentes, **caractérisée en ce que** le ou les alcanes linéaires volatils sont choisis parmi le n-heptane (C7), le n-octane (C8), le n-nonane (C9), le n-décane (C10), le n-undécane (C11), le n-dodécane (C12), le n-tridécane (C13), le n-tétradecane (C14), et leurs mélanges.

4. Composition cosmétique selon la revendication 3, **caractérisée en ce que** le ou les alcanes linéaires volatils sont choisis parmi le n-nonane, le n-undécane, le n-dodécane, le n-tridécane, le n-tétradécane, et leurs mélanges.

5. Composition cosmétique selon l'une quelconque des revendications précédentes, **caractérisée en ce que** le ou les alcanes linéaires volatils sont d'origine végétale.

6. Composition cosmétique selon l'une quelconque des revendications précédentes, **caractérisée en ce que** le ou les alcanes linéaires volatils sont présents en une teneur allant de 2,5 à 90% en poids, de préférence de 2,6 à 50% en poids, encore plus préférentiellement de 3 à 40% en poids, et mieux de 3 à 30% par rapport au poids total de la composition.

7. Composition cosmétique selon l'une quelconque des revendications précédentes, **caractérisée en ce que** le ou les tensioactifs cationiques sont choisis parmi les amines grasses primaires, secondaires ou tertiaires, éventuellement polyoxyalkylénées, ou leurs sels, les sels d'ammonium quaternaire, et leurs mélanges.

8. Composition cosmétique selon la revendication précédente, **caractérisée en ce que** les sels d'ammonium quaternaires sont choisis parmi :
- les sels d'ammonium quaternaires répondant à la formule générale (I) suivante : 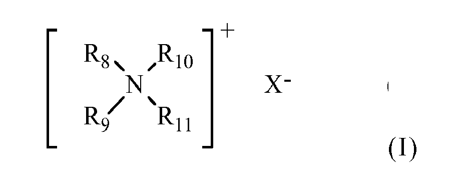 dans laquelle les radicaux R₈ à R₁₁, identiques ou différents, représentent un radical aliphatique, linéaire ou ramifié, comportant de 1 à 30 atomes de carbone et pouvant comporter des hétéroatomes, ou bien un radical aromatique, au moins un des radicaux R₈ à R₁₁ comportant de 8 à 30 atomes de carbones ;
- les sels d'ammonium quaternaire de l'imidazoline répondant à la formule (II) suivante : 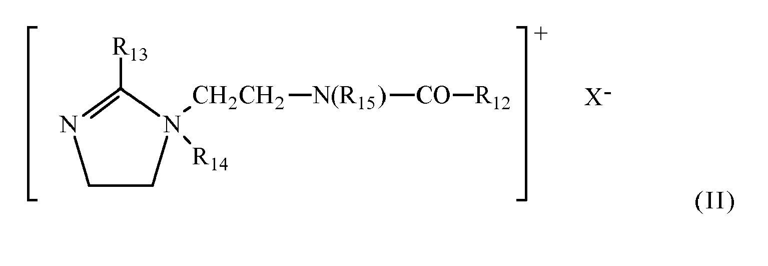 dans laquelle R₁₂ représente un radical alcényle ou alkyle comportant de 8 à 30 atomes de carbone, R₁₃ représente un atome d'hydrogène, un radical alkyle en C₁-C₄ ou un radical alcényle ou alkyle comportant de 8 à 30 atomes de carbone, R₁₄ représente un radical alkyle en C₁-C₄, R₁₅ représente un atome d'hydrogène, un radical alkyle en C₁-C₄, X⁻ est un anion choisi dans le groupe des halogénures, phosphates, acétates, lactates, alkylsulfates, alkyl- ou alkylaryl-sulfonates ;
- les sels de diammonium quaternaire de formule (III) : 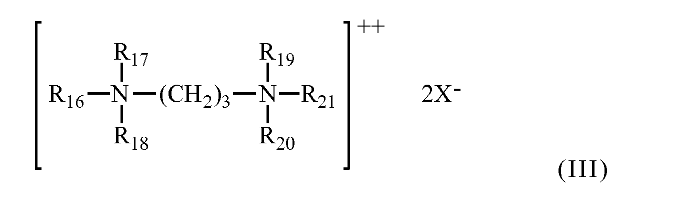 dans laquelle R₁₆ désigne un radical aliphatique comportant de 16 à 30 atomes de carbone, R₁₇, R₁₈, R₁₉, R₂₀ et R₂₁, identiques ou différents, sont choisis parmi l'hydrogène ou un radical alkyle comportant de 1 à 4 atomes de carbone, et X⁻ est un anion choisi dans le groupe des halogénures, acétates, phosphates, nitrates et méthylsulfates ;
- les sels d'ammonium quaternaire contenant au moins une fonction ester et répondant à la formule (IV) suivante : 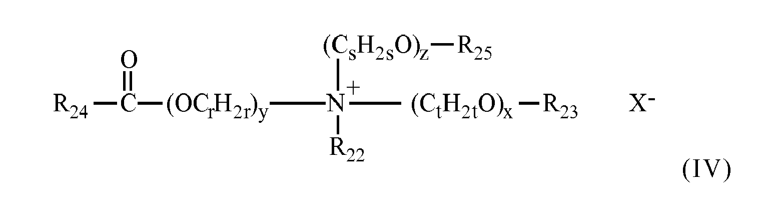 dans laquelle :
R₂₂ est choisi parmi les radicaux alkyles en C₁-C₆ linéaires ou ramifiés et les radicaux hydroxyalkyles ou dihydroxyalkyles en C₁-C₆ ;
R₂₃ est choisi parmi :
- le radical 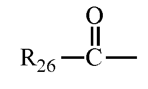
- les radicaux R₂₇ qui sont des radicaux hydrocarbonés en C₁-C₂₂, linéaires ou ramifiés, saturés ou insaturés,
- l'atome d'hydrogène,
R₂₅ est choisi parmi :
- le radical 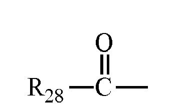
- les radicaux R₂₉ qui sont des radicaux hydrocarbonés en C₁-C₆, linéaires ou ramifiés, saturés ou insaturés,
- l'atome d'hydrogène,
R₂₄, R₂₆ et R₂₈, identiques ou différents, sont choisis parmi les radicaux hydrocarbonés en C₇-C₂₁, linéaires ou ramifiés, saturés ou
insaturés ;
r, s et t, identiques ou différents, sont des entiers valant de 2 à 6 ;
y est un entier valant de 1 à 10 ;
x et z, identiques ou différents, sont des entiers valant de 0 à 10 ;
X⁻ est un anion simple ou complexe, organique ou inorganique ;
sous réserve que la somme x + y + z vaut de 1 à 15, que lorsque x vaut 0 alors R₂₃ désigne R₂₇ et que lorsque z vaut 0 alors R₂₅ désigne R₂₉.

9. Composition cosmétique selon l'une quelconque des revendications précédentes, **caractérisée en ce que** le ou les tensioactifs cationiques sont présents en une teneur allant de 0,1 à 10% en poids, de préférence de 0,5 à 8% en poids, et mieux encore de 1 à 5% en poids, par rapport au poids total de la composition.

10. Composition cosmétique selon l'une quelconque des revendications précédentes, **caractérisée en ce que** le rapport pondéral de la quantité d'alcane(s) linéaire(s) volatil(s) sur la quantité de tensioactif(s) cationique(s) varie de 0,01 à 1,5, de préférence de 0,1 à 1,4, et mieux encore de 0,5 à 1,2.

11. Composition cosmétique selon l'une quelconque des revendications précédentes, **caractérisée en ce qu'**elle comprend en outre au moins une silicone et/ou au moins un alcool gras en C₈-C₄₀ et/ou au moins un polymère cationique.

12. Composition cosmétique selon l'une quelconque des revendications précédentes, **caractérisée en ce qu'**elle comprend en outre au moins un additif, différent des composés tels que définis dans l'une quelconque des revendications 1 à 11, et choisi parmi des polymères associatifs ou non, ioniques ou non-ioniques, des silanes, des tensioactifs anioniques, des tensioactifs amphotères ou zwittérioniques, des tensioactifs non ioniques, des protéines, des vitamines, des agents réducteurs, des plastifiants, des adoucissants, des agents anti-mousse, des agents hydratants, des pigments, des argiles, des charges minérales, des filtres UV, des colloïdes minéraux, des peptisants, des solubilisants, des parfums, des conservateurs, des agents nacrants, des propulseurs, et des épaississants minéraux ou organiques.

13. Utilisation de la composition cosmétique selon l'une quelconque des revendications précédentes, pour le traitement des matières kératiniques, de préférence des cheveux.

14. Procédé de traitement cosmétique des matières kératiniques, de préférence des fibres kératiniques, et mieux encore des cheveux, comprenant l'application de la composition cosmétique selon l'une quelconque des revendications 1 à 12.
